# EUROPEAN PATENT APPLICATION

(11) **EP 2 610 001 A1**
(43) Date of publication of application: **03.07.2013**
(21) Application number: 11195817.9
(22) Date of filing: 27.12.2011
(51) Int. Cl.: B01J 8/00, B01J 8/04, C01C 1/04, C07C 29/152

(54) **Adiabatic multi-bed catalytic converter with inter-bed cooling and a related process**

(71) Applicant: Ammonia Casale S.A., 6900 Lugano-Besso (CH)
(72) Inventor: Filippi, Ermanno, 6976 Castagnola (CH)
(74) Representative: Zardi, Marco

(57) **Abstract**

A process for catalytic conversion with multiple catalytic beds and inter-bed cooling of process streams, where at least one process stream from a generic first catalytic bed is first cooled by passage in a heat exchanger, and then is quenched by mixing with a flow rate of fresh reagents; a converter is also disclosed comprising at least one quench line (109) arranged to feed an amount of fresh reagents (31) in at least one mixing chamber (108) located between two adjoining catalytic beds (11, 12).

## Description

### Field of the invention

The present invention relates to an adiabatic multi-bed catalytic converter with inter-bed cooling, and to a process for catalytic conversion comprising a reaction steps in a plurality of catalytic beds, and inter-bed cooling of the process stream passing from one bed to another.

### Prior Art

An adiabatic multi-bed catalytic converter is understood in this description as a chemical reactor comprising a plurality of catalytic beds arranged in series, where the chemical reaction occurring in each bed is substantially adiabatic, i.e. without heat exchange means immersed in the bed. Multi-bed converters of this kind are used for example for synthesis of ammonia or methanol. Each bed can also be defined a stage of the converter.

A gaseous charge is fed to the first bed and generates a first and partially reacted product stream, which is passed to a second bed, and so on. As the reaction stages are substantially adiabatic, the temperature of the process stream may significant increase while passing through each stage and an excessive temperature may damage a subsequent bed or slow down the chemical reaction. Hence it is known to provide inter-bed (or inter-stage) cooling. Inter-bed cooling has the advantage that the process stream inlet temperature of a subsequent bed can be brought to a desired value and that useful heat can be recovered.

The prior art include quench converters and converters with inter-bed heat exchangers.

A quench converter is disclosed in US-A-4,372,920 and provides that the product stream exiting a catalytic bed is cooled by mixing with a stream of fresh reagents (quench gas). The flow rate of the quench gas is determined in order to bring the resulting process stream/quench gas mixture to a desired inlet temperature for the next bed. This arrangement is simple but has a disadvantage in that some reagents bypass one or more catalytic beds. For example a quench gas stream injected downstream the first bed will bypass said first bed. Hence, considering that the amount of quench gas can be 30 to 50 % of the total flow entering the reactor, the overall efficiency of conversion is significantly affected.

A converter with inter-bed heat exchangers is disclosed in US 4,769,220.The hot process stream exiting a catalytic bed is cooled by a passage in a hot side of an indirect heat exchanger, for example a bundle of heat exchange tubes, arranged between said bed and a subsequent bed of the converter. The cold side of the heat exchanger is usually fed with the stream of the fresh reagents, in order to recover heat and pre-heat the reagents. Hence the fresh reagents are progressively pre-heated while passing through the cold side of a first and subsequent inter-bed heat exchangers. Furthermore, the temperature of a preheated fresh gas at the entrance of a second or further heat exchanger can be adjusted with a by-pass injection of cold (non-preheated) fresh gas. A converter with inter-bed heat exchanger and by-pass gas injection is disclosed in US 5,135,722.

A converter with inter-bed heat exchangers has an advantage, compared to a quench reactor, in that the full amount of fresh charge passes through all the available catalytic beds. However the converter is significantly more expensive due to the need of said heat exchangers. In particular the aforesaid by-pass gas injection requires a sophisticated design because the gas need be injected between two of the heat exchangers. For example in some embodiments the by-pass gas injection requires a by-pass gas feeding tube to pass through one or more tube sheets with related problems such as sealing and different thermal elongation. These problems can be solved for example with labyrinth seals which, however, are expensive.

It is to be noted that above prior-art designs become unpractical with more than three reaction stages and related two inter-bed cooling stages. A quench converter with more than three stages would suffer a too large amount of fresh gas bypassing one or more reaction stages for quenching, therefore jeopardizing the advantage of having more reaction stages; a converter with inter-bed heat exchanger would be too complicated and expensive.

### Summary of the invention

The invention is aimed to overcome the above drawbacks of multi-bed converters.

This aim is accomplished with a process for catalytic conversion and with a multi-bed catalytic converter according to the independent claims.

A process for catalytic conversion with a plurality of adiabatic reaction steps in a series of catalytic beds and inter-bed cooling is characterized in that at least one process stream from a generic first catalytic bed is mixed with a quench flow of fresh reagents, said reagents being at a lower temperature than said process stream, before the process stream enters a second and downstream catalytic bed.

Said mixing with the quench flow take place preferably after a step of inter-bed cooling between said first and second bed, and said fresh reagents have a temperature lower than the temperature of the process stream after said inter-bed cooling. Hence, the inter-bed cooling provides a first cooling of the process stream, and the mixing with said quench flow provides a second and further cooling of the process stream, before admission in said second bed.

A converter according to the invention comprises a plurality of catalytic beds arranged in a series, so that a process stream exiting a first or intermediate catalytic bed is fed to a next catalytic bed, and a process stream exiting a last catalytic bed is passed to a product stream outlet, and a plurality of inter-bed heat exchangers arranged to cool a process stream flowing from one bed to another, and is characterized by comprising at least one quench line arranged to feed an amount of fresh reagents in at least one mixing chamber located between two adjoining catalytic beds.

Preferably said chamber is located to receive a process stream from the inter-bed exchanger between said two adjoining beds, and is in communication with the inlet of the downstream bed of said two adjoining beds, so that a process stream from said upstream bed and after cooling in the inter-bed exchanger is mixed with said amount of fresh reagents in said mixing chamber, and resulting mixture enters said downstream bed.

The mixing of process stream with a quench flow rate of fresh reagents takes place before entrance of a process stream into one selected catalytic bed, or before entrance into more or each catalytic beds, according to various embodiments. Hence said mixing may take place in one or more points. It shall be noted that the term of "generic" first catalytic bed denotes the first (upstream) of two adjoining beds; said generic first bed may equally denote the first catalytic bed of the series, namely the bed receiving the fresh charge of reagents, or a subsequent bed of the series.

In the point or points where the injection of quench gas is performed, an inter-bed cooling duty is accomplished in part by the respective heat exchanger and in part by the mixing with the quench flow. The inter-bed cooling duty may be defined as a temperature drop between the hot process stream leaving a catalytic bed, and the colder process stream entering a subsequent catalytic bed. Preferably, a major part (more than 50%) of said inter-bed cooling duty is accomplished by the inter-bed heat exchanger, and a minor part (less than 50%) is accomplished by the quenching. More preferably at least 70%, and more preferably around 80% of the cooling duty is accomplished by inter-bed heat exchangers. The converter is preferably designed in accordance. A related advantage is that the bypass rate, i.e. the amount of fresh reagents bypassing one or more beds, is reduced. As a result, the inter-bed heat exchangers will be smaller, the mechanical construction will be simpler, as explained later, and it is possible to build a converter with four or more catalytic beds, obtaining a better conversion per pass.

The apportionment of the cooling duty between the heat exchanger and the quenching may gradually change according to the age of the catalyst. It is well known in fact that ageing of the catalyst results in decrease of the activity of the catalyst. For this reason, the optimal operating temperature of the catalyst may vary according to the age of catalyst, and therefore the amount of quench flow may change accordingly. The above preferred apportionment of the cooling duty, with more than 50% of the temperature drop across the inter-bed heat exchanger, may apply only for some conditions of catalyst age.

The invention provides, in other words, a hybrid converter and process, where a part of the inter-bed cooling duty is accomplished by inter-bed heat exchangers, and a remaining part of said cooling duty is accomplished by mixing the process stream with fresh reagents.

For example, the process stream exiting the first bed is first cooled by passing through a first heat exchanger, and then is further cooled in a mixing chamber, by mixing with a rate of fresh reagents; the cooled mixture now including the process stream partially reacted in the first bed, and the quench rate of fresh reagents, enters the second bed where it is further reacted. The inlet temperature of the second bed can be controlled in accurate manner.

In other embodiments, the quenching/mixing may take place in several points, namely downstream several or each of the catalytic beds.

For example, in a three-bed converter with two inter-bed exchangers it is preferred that quenching/mixing takes place at least downstream the first bed and related inter-bed heat exchanger, namely before entrance of the process stream into the second bed, because the temperature is the highest in this point. This however shall not be construed as a limitation.

The injection of one or more quench stream(s) allows a precise control of the temperature of the process stream but, in accordance with a preferred feature, a greater amount of heat is removed during the previous passage of the process stream through a heat exchanger, and only a minor amount of heat removal is effected with the injection of the quench stream(s). Hence the flow rate of reactants bypassing one or more catalytic beds, for use as quench streams, is reduced and the negative effect of said bypass on the efficiency is also reduced. On the other hand there is no need to inject the quench gas directly in the heat exchangers, because the quenching / mixing takes place downstream the inter-bed exchangers, and for example in a mixing room located between two adjoining beds.

The invention has advantages in that it achieves cooling of the process stream and accurate control of the inlet temperature of the catalytic beds, avoiding the disadvantage of quench reactors, namely the relatively large bypass rate, and avoiding as well the complicated design of reactors with a direct gas injection within tubes of a heat exchanger. A significant advantage of the invention is that it makes affordable to make converters with more than three catalytic beds and two inter-bed coolers, for example five beds and four inter-bed heat exchangers. The prior art is substantially restricted to a maximum of three beds/two inter-bed exchangers, because otherwise the efficiency would drop due to large bypass rate (quench reactors) or the converter will be too expensive because of complex internals. Hence the invention can be used to realize converters with several catalytic beds obtaining a higher conversion efficiency than in the prior art.

A further preferred feature of the invention is the following. It has been noted that in a converter with more than three beds (e.g. four- or five-beds converter) it may be preferred to cool the process stream leaving the first bed of the converter only by means of quenching, that is, to remove the first inter-bed heat exchanger, and then the combined cooling by means of inter-bed heat exchange (in a indirect heat exchanger) and quenching/mixing is carried out on the process stream leaving the second bed and all or at least some of the following beds.

Another aspect of the invention is a method for controlling the inlet temperature of a process stream fed to a catalytic bed in an adiabatic multi-bed catalytic converter. Said process stream, after leaving an upstream catalytic bed and preferably also after leaving an inter-bed heat exchanger, is mixed with a flow of colder fresh reagents. Said flow of colder reagents is adjusted in order to obtain a desired temperature of the mixed flow entering said catalytic bed.

The advantages of the invention will be elucidated with the help of the following description of preferred and non-limiting embodiments.

### Brief description of the drawings

Fig. 1 is a block diagram showing a preferred embodiment of the invention.
Fig. 2 is a sketch of a converter according to a preferred embodiment.

### Detailed description of preferred embodiments

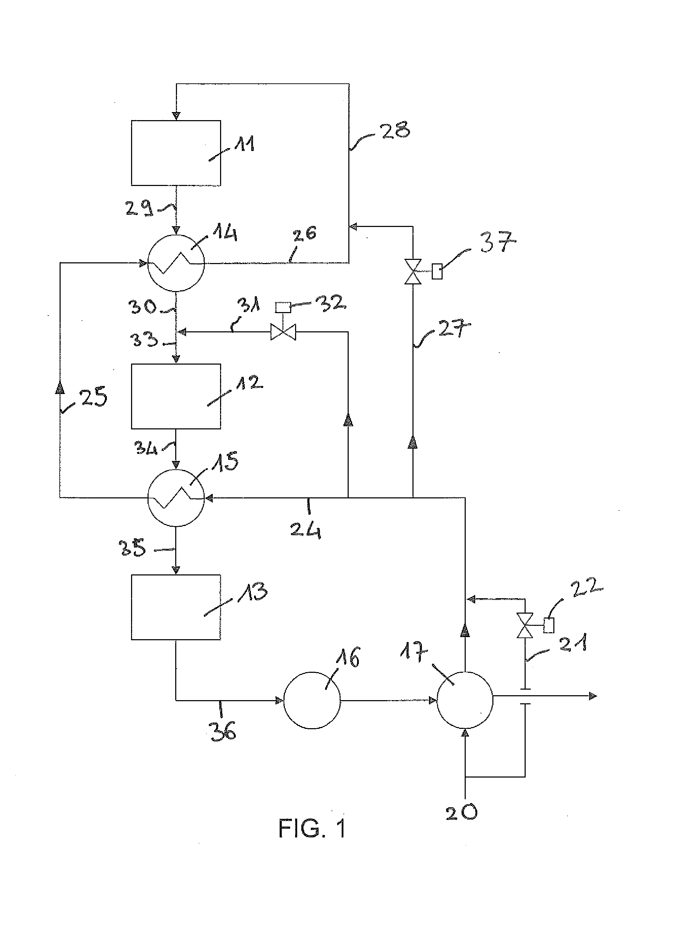

Fig. 1 is a block diagram of a process with three catalytic beds 11, 12, 13 and two inter-bed heat exchangers 14, 15.

Reference 23 denotes a flow of gaseous reactants which is directed to conversion. Said flow 23 preferably undergoes a preliminary heating: for example Fig. 1 shows an embodiment where reagents enter as stream 20 and undergo preliminary heating in a heat recovery exchanger 17, with a by-pass line 21 for temperature control. Flow rate in said by-pass line 21 is controlled with a valve 22.

A first portion of said flow 23, denoted as stream 24, is pre-heated by passage through the inter-bed exchangers 15 and 14, thus obtaining a relatively hot stream 26, which can be mixed with a second portion of said flow 23, coming from a bypass line 27 and bypassing the inter-bed exchangers, for control of the first bed inlet temperature in stream 28.

More in detail, the stream 24 is passed through the heat exchanger 15, thus cooling the process stream from the second bed 12 while being heated to form a partially pre-heated gas stream 25. This gas stream 25 is then passed through the inter-bed exchanger 14, cooling the process stream leaving the first bed 11 and obtaining said stream 26.

The passage of the reactants 28 through the first bed 11 produces a first process stream 29. Said stream 29 usually contains some products and some reagents which will be converted in the following beds. The cooled process stream 30 at the outlet of the heat exchanger 14 is mixed with a quench stream 31 of fresh reagents at a lower temperature, in order to precisely adjust the temperature of the stream entering the next catalytic bed 12.

Said quench stream 31 is taken from the feed directed to conversion and before passage in the inter-bed exchangers, i.e. said quench stream is a third portion of said flow 23, as shown in Fig. 1.

It may be noted that quench stream 31 bypasses the first bed 11. More in general, a quench stream will bypass one or more catalytic beds. Hence, it is preferred to take the quench stream(s) from a relatively cold point so that, for a given quenching effect, the amount of reagents bypassing one or more catalytic beds will be small.

In order to achieve optimum conversion in the second bed 12, a certain temperature drop of the hot stream 29 leaving the first bed 11 is desired. In a preferred embodiment, the major part of this temperature drop is given by the passage through the heat exchanger 14, and only a minor part of said drop is given by the direct mixing of the process stream 30, leaving the heat exchanger 14, with the colder stream 31. The same is applicable to other quench lines, if more than one quench lines are provided.

For example, if desired inlet temperature of second bed 12 is 100 °C less than outlet temperature of first bed 11, a first ΔT of 80°C is preferably given by the passage through exchanger 14, and only a remaining ΔT of 20°C is given by mixing/quenching with the quench stream 31 (ΔT stands for difference of temperature).

The flow rate (m3/s or kg/s) of the quench stream 31 is regulated with at least one valve 32, in order to obtain a desired temperature of the resulting process stream 33, now including the process stream 30 and the amount of fresh reactants added via line 31, entering the second bed 12.

In second bed 12, the chemical conversion progresses and a process stream 34 is produced. Said process stream 34 is cooled in the second inter-bed exchanger 15, and cooled stream 35 enters the third bed 13 to complete conversion. The product stream 36 leaving the third bed is then cooled in heat exchangers 16 and 17.

In the embodiment of Fig. 1 the only quench line is line 31 added downstream the first inter-bed exchanger 14; in further embodiments, however, other quench lines may be provided. For example, still referring to Fig. 1, another quench line may add a flow of fresh reactants to the process stream 35 in order to control the inlet temperature of the third bed 13. In some embodiments (not shown) the mixing can take place upstream the inter-bed exchanger, for example referring to Fig. 1 the quench stream 31 can merge with process stream 29.

According to a further preferred feature, a quench stream is added at least before entrance in the second bed of the series, such as bed 12 in Fig. 1. In fact, the risk of overheating is higher through the first bed, where the feed is more reactive.

It should be noted that the flow rate in the bypass line 27 and the flow rate in the quench line 31 can be regulated in an independent manner by means of valves 37 and 32 respectively. Hence the invention introduces a further degree of freedom in the regulation of the temperature of the evolving process streams. In particular, in the example of Fig. 1, the inlet temperature of beds 11 (stream 28) and bed 12 (stream 33) can be adjusted in a precise manner.

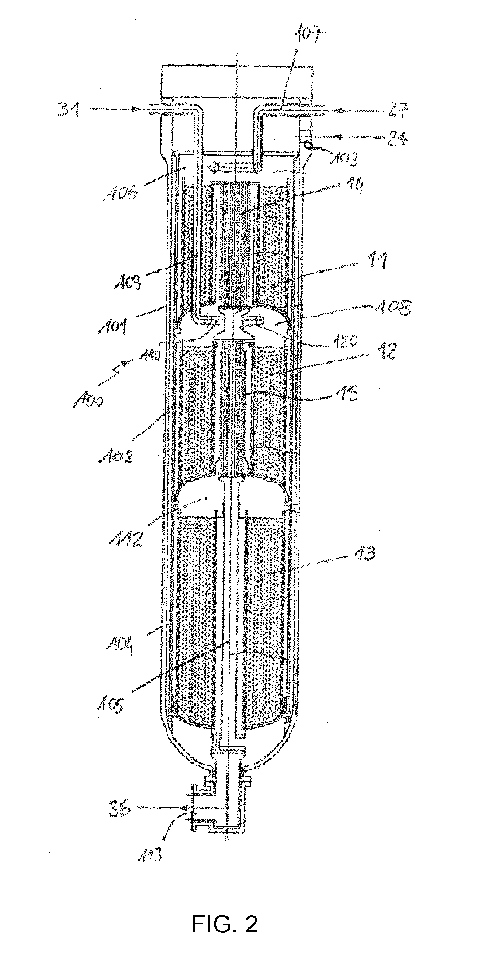

Fig. 2 shows a converter (or reactor) 100 implementing the process of Fig. 1. The converter 100 has a shell 101 containing a basket 102 for supporting the three beds 11, 12 and 13. The converter 100 has an inlet 103 for the fresh charge of reactants and an outlet 113 for a product stream.

In this embodiment the catalytic beds have an annular structure and the inter-bed exchangers 14, 15 have the form of bundles of tubes coaxial with the annular beds 11 and 12. Hence the inside of tubes is the cold side of the exchangers 14 and 15, traversed by a cooling medium which, in this embodiment, is given by the fresh reactants; the outside of the tubes is a hot side traversed by the process stream leaving the bed 11 or 12, respectively.

The stream 24 of reactant gas enters an inlet 103 and flows through an interspace 104 between the shell and cartridge, and then through an upcomer 105 and through the tube side (i.e. inside tubes) of the inter-bed exchangers 15 and 14, acting as a cooling medium. In a top chamber 106 the pre-heated reactants (stream 26 in Fig. 1) are mixed with the bypass stream 27, added via a distributor 107. The reactants then enter the first bed 11, flowing radially through the shell side (i.e. outside of tubes) of the first inter-bed exchanger 14.

The bed 11 is contained in a basket with perforated side walls, allowing the flow of the process stream, according to technique which is known and need not be described.

The product stream leaving the hot side of first inter-bed exchanger 14 enters a mixing chamber 108. Said mixing chamber 108 is in communication with the entrance of the second catalytic bed 12. In this chamber 108, the process stream coming from the exchanger 14 (denoted as 30 in Fig. 1) is merged with the quench stream 31 coming via a quench feeder 109.

As seen in Fig. 2, said quench feeder 109 passes through the annular bed 11 and through the lower wall supporting the catalyst, but it does not affect the design of the inter-bed exchanger 14. In particular, said feeder 109 need not pass through plate sheets of said exchanger 14 and the design of said exchanger 14 is substantially unaffected.

In a preferred embodiment, said quench feeder 109 ends with a distributor 110. More preferably distributor 110 is a toroidal distributor around a collector 120 for connection of the tube side of exchanger 14 to the tube side of exchanger 15, i.e. said collector 120 leads the gas from the tubes of the second exchanger 15 to the tubes of the first and hotter exchanger 14.

Another quench line (not shown) could be directed to the mixing chamber 112 between the second bed and third bed, according to other possible embodiments.

Fig. 2 allows further appreciation of the advantages of the invention. The addition of quench stream 31 via line 109 allows an accurate control of the temperature at inlet of the second bed 12, without a complex and/or expensive design of the converter.

### EXAMPLE

The following table discloses a comparison of different set-ups for an ammonia synthesis converter, according to the prior art and according to the invention.

| Converter | | (A) | (B) | (C) | (D) |
|---|---|---|---|---|---|
| | | 3 beds, 2 quenches | 3 beds, 2 interchangers | 3 beds, 2 interchangers, 1 quench | 5 beds, 4 interchangers, 3 quenches |
| | | (prior art) | (prior art) | | |
| NH3 in | % mol | 2.6 | 2.6 | 2.6 | 2.6 |
| NH3 out | % mol | 15.4 | 18.2 | 18.1 | 19.0 |

All variables and conditions are supposed equal for all four converters type, i.e. the catalyst volume, the production rate, the converter inlet composition and temperature and the operating pressure, so that to have a correct comparison.

Case (A) relates to a converter according to the state of the art where the gas leaving the first bed and second bed is cooled only by heat exchangers, and where the cooling gas is the incoming fresh feed gas.

Case (B) relates to a converter according to the state of the art, where the gas leaving the first bed and second bed is cooled only by quenching, i.e. by mixing the hot gas exiting the bed with fresh incoming feed gas.

Case (C) relates to a converter according to an embodiment of the invention, where the gas leaving the first bed and second bed is cooled only by heat exchangers, and where the cooling gas is the incoming fresh feed gas, such as stream 24 of Figs 1 and 2. In addition, at the exit of the hot side of the heat exchanger downstream bed 1, the gas is further mixed (quenched) with fresh incoming feed gas, such as stream 31 of Figs. 1 and 2.

Case (D) relates to a converter according to another embodiment of the invention where the number of the catalytic beds is increased to five, and after the first three inter beds heat exchangers the gas is further quenched to control finally its temperature before entering the following bed.

The table shows that the 3-beds, 2-quenches converter of case (A) is at a disadvantage with respect to the 3-beds, 2-interchangers converter (B), due to the by-pass effect of the quench gas. The inventive 3-beds, 2-interchanger embodiment with one quench (case C) has almost the same conversion per pass of the 3-bed, 2-interchanger case (B), although its design is significantly simpler thanks to the presence of the quench line. Hence the invention has an advantage in that the same conversion per pass is obtained at a smaller capital cost for the equipments.

Finally the case (D) with five beds, four inter-bed exchangers and three quenches has a significant advantage of conversion per pass over the 3-beds, 2-interchangers converter, thanks to the higher number of reaction stages. This high number of stages would be very difficult and expensive to obtain with a conventional design.

## Claims

1. A process for catalytic conversion of a flow of reagents into a product stream, comprising a plurality of adiabatic reaction steps though respective catalytic beds (11, 12, 13) arranged in a series, so that a process stream exiting the first bed or an intermediate catalytic bed is fed to a next catalytic bed, and a process stream exiting the last catalytic bed forms the aforesaid product stream, and comprising inter-bed cooling steps (14, 15) of the process streams flowing from one bed to another, where said inter-bed cooling steps provide that a process stream is cooled by indirect heat exchange with a cooling medium, the process being **characterized in that**:
at least one process stream (30), leaving a generic first catalytic bed (11) for passage into a second and downstream catalytic bed, is mixed with a quench flow of reagents (31) before entering said second bed, said quench flow (31) having a temperature lower than temperature of said process stream (30).

2. A process according to claim 1, **characterized in that** the mixing of said process stream (30) and quench flow (31) takes place after a respective step of inter-bed cooling of the process stream (30) between said first catalytic bed and said second catalytic bed, and said quench flow (31) has a temperature lower than the temperature of the process stream after said step of inter-bed cooling.

3. A process according to claim 1 or 2, wherein a step of inter-bed cooling and said mixing with a quench flow provide together a temperature drop of a process stream passing from the outlet of said first bed to the inlet of said second bed, and wherein more than 50% and preferably at least 80% of said temperature drop is given by said step of inter-bed cooling.

4. A process according to any of the preceding claims, where each process stream passing from a catalytic bed to a following catalytic bed of said series of catalytic beds, is mixed with a respective quench flow of reagents.

5. A process according to any of claims 1 to 4, where the flow rate of the quench flow or flow rates of each of the quench flows is/are regulated in order to adjust the process stream inlet temperature of respective catalytic bed or beds.

6. A process according to any of previous claims, said cooling medium for inter-bed cooling being a flow of fresh reagents.

7. A process according to any of previous claims, for the synthesis of ammonia or for the synthesis of methanol.

8. Adiabatic multi-bed catalytic converter (100) comprising:
- a shell (101) including at least an inlet (103) for a stream of fresh reagents and an outlet (113) for a product stream,
- a plurality of catalytic beds (11, 12, 13) contained in said shell,
- said catalytic beds being arranged in a series so that a process stream exiting a first or intermediate catalytic bed is fed to a next catalytic bed, and a process stream exiting a last catalytic bed is passed to said product stream outlet,
- a plurality of inter-bed heat exchangers (14, 15) fed with a cooling medium and arranged to cool a process stream flowing from one bed to another,
**characterized in that**:
- said converter comprises at least one quench line (109) arranged to feed an amount of fresh reagents (31) in at least one mixing chamber (108) located between two adjoining catalytic beds (11, 12).

9. A converter according to claim 8, said chamber being located to receive a process stream leaving the inter-bed exchanger (14) between said two adjoining beds, and being in communication with the inlet of the downstream bed (12) of said two adjoining beds.

10. A converter according to claim 9, where:
- a first and upstream catalytic bed of said two adjoining catalytic beds has an annular shape and the respective inter-bed heat exchanger is a tube heat exchanger coaxial with said first catalytic bed,
- the tubes of said heat exchanger are fed with said cooling medium thus forming a cold side of said heat exchanger, and the outside of said tubes form a hot side of the heat exchanger receiving the hot process stream leaving said first catalytic bed, and
- said hot side of the heat exchanger is also in communication with said mixing chamber (108), so that cooled process stream leaving said cold side enters said mixing chamber.

11. A converter according to any of claims 8 to 10, comprising more than three catalytic beds and more than two inter-bed heat exchangers, and preferably comprising five catalytic beds.

12. A converter according to any of claims 8 to 11, for use as methanol or ammonia converter.

13. A method for controlling the inlet temperature of a process stream fed to a catalytic bed in an adiabatic multi-bed catalytic converter, wherein said process stream, after leaving an upstream catalytic bed and preferably after a first cooling by means of a passage in an inter-bed heat exchanger, is mixed with a flow of colder fresh reagents, and said flow of colder reagents is adjusted in order to obtain a desired temperature of the mixed flow entering said catalytic bed.
